# EUROPEAN PATENT APPLICATION

(11) **EP 4 494 545 A1**
(43) Date of publication of application: **22.01.2025**
(21) Application number: 22932408.2
(22) Date of filing: 01.12.2022
(51) Int. Cl.: A61B 5/00, A61B 5/11, A61B 7/04

(54) **METHOD FOR PROVIDING FETUS MONITORING SERVICE AND SERVER FOR PERFORMING SAME**

(30) Priority: 16.03.2022 KR 20220033004
(71) Applicant: Sungkwang Medical Foundation, Seoul 06135 (KR)
(72) Inventor: KIM, Min Young, Seoul 06667 (KR)
(74) Representative: WSL Patentanwälte Partnerschaft mbB
(86) International application number: PCT/KR2022/019338
(87) International publication number: WO 2023/177041

(57) **Abstract**

An embodiment of the present disclosure provides a method of providing a fetal monitoring service, including obtaining, from a monitoring device in time series, raw data including movement data related to a fetus and a pregnant woman and acoustic data related to the fetus and a surrounding stimulus, removing noise from the raw data by using time series information and generating valid data by matching the noise-removed raw data with pre-stored user information, determining a health status of the fetus by using the valid data and generating result data, and providing the result data to a user terminal.

## Description

### Technical Field

Embodiments of the present disclosure relate to a method of providing a fetal monitoring service and a server for performing the method.

### Background Art

With the progress of aging society, interest in health has increased significantly, and with the development of electronic communication technology, demands for technology for monitoring a health status at all times by using a mobile device, a personal computer, and a wearable device have increased. Currently, various services specialized in health management for people with chronic diseases, such as diabetes and high blood pressure, have been developed and provided as a health care service using a mobile device.

Meanwhile, low birth rates and aging society have led to aging of a childbirth age, and as a result, improving health of pregnant women and fetuses has become important. In particular, in a case of high-risk pregnancy, there is a high possibility that health of a pregnant woman and fetus will be at risk, and thus, continuous monitoring and health care are required during the pregnancy. Health care for pregnant women and fetuses is more useful when the health care is continuous and is able to be performed in daily life, but there are limits to self-management of pregnant women and there is currently lack of services specialized in monitoring health statuses of pregnant women and fetuses by using mobile devices or wearable devices.

Fetal movement is one of key factors for determining a health status of a fetus, and many technologies have been developed to measure the fetal movement. Currently, there are measurement methods using ultrasound and magnetic resonance imaging (MRI) as methods for clinically measuring a position, a posture, and movements of a fetus, but both methods are expensive and are able to be performed only for a short period of time. Kick-counting measurement method, in which a pregnant woman personally detects and records fetal movement every day, may also be considered. However, the fetal movement detected by the pregnant woman is very subjective and it is difficult to quickly detect changes such as a decrease in movement.

In this regard, in the related art, a pregnant woman has to visit a hospital periodically and receive ultrasound examinations or medical examinations to determine a status of a fetus, but there is a limit to providing an appropriate service because the amount of information is insufficient to monitor various fetal changes through short-time observation during a hospital visit.

### Disclosure

### Technical Problem

The present disclosure is for solving the above problems and/or limits, and provides a method of providing a fetal monitoring service, wherein a health status of a fetus is monitored non-invasively and continuously without interfering with a daily life of a pregnant woman, and a server for performing the method.

In addition, the present disclosure provides, to a pregnant woman through a monitoring service, a method of taking an appropriate measure, such as an exercise program, and provides an effective health management service without increasing the frequency of hospital visits.

However, such problems are only examples and the scope of the present disclosure is not limited thereby.

### Technical Solution

An embodiment of the present disclosure provides a method of providing a fetal monitoring service, including obtaining, from a monitoring device in time series, raw data including a position, a posture, and movement data related to a fetus and a pregnant woman and acoustic data related to the fetus and a surrounding stimulus, removing noise from the raw data by using time series information and generating valid data by matching the noise-removed raw data with pre-stored user information, determining a health status of the fetus by using the valid data and generating result data, and providing the result data to a user terminal.

According to an embodiment of the present disclosure, the acoustic data may include first acoustic data related to the fetus and second acoustic data related to ambient sound excluding the fetus.

According to an embodiment of the present disclosure, the determining of the health status of the fetus and the generating of the result data may include determining the health status of the fetus by linking, in time series, the movement data, the first acoustic data, and the second acoustic data.

According to an embodiment of the present disclosure, the movement data may include acceleration data sensed by an acceleration sensor of the monitoring device.

According to an embodiment of the present disclosure, the user information may include personal information of the pregnant woman and status information of the pregnant woman.

According to an embodiment of the present disclosure provides a computer-readable recording medium storing a program for executing the above method.

According to an embodiment of the present disclosure provides a service providing server including a memory storing at least one program, a communication unit configured to transmit and receive data to and from a monitoring device for measuring a status of a fetus, a user terminal, and a medical system server, and at least one processor configured to provide a fetal monitoring service to a user by executing the at least one program, wherein the memory includes instructions that cause the at least one processor to obtain, from the monitoring device in time series, raw data including movement data related to a fetus and a pregnant woman and acoustic data related to the fetus and a surrounding stimulus, remove noise from the raw data by using time series information and generate valid data by matching the noise-removed raw data with pre-stored user information, determine a health status of the fetus by using the valid data and generate result data, and provide the result data to the user terminal.

Other aspects, features, and advantages may become clear from the following drawings, the claims, and the detailed description of the present disclosure.

### Advantageous Effects

According to the above-described technical solutions of the present disclosure, embodiments of the present disclosure may provide a service for managing and improving health of a pregnant woman and fetus by providing a service for monitoring a status of the fetus, and increase user convenience.

In addition, embodiments of the present disclosure may provide a service for, by monitoring a status of a fetus, predicting torticollis, head asymmetry, and scoliosis, predicting a genomic abnormality such as various types of muscle disease, motor neuron disease, and chromosomal disease, and discovering, in early stages, a cranial nerve function status through prediction of cerebral palsy, developmental delay, intellectual disability, and autism. In addition, embodiments of the present disclosure may provide a service for providing information necessary to make an appropriate decision on a cesarean section by predicting the degree of risk of childbirth, and may promote health of a pregnant woman and fetus throughout the entire period of pregnancy and childbirth.

Obviously, the scope of the present disclosure is not limited by such effects.

### Description of Drawings

FIG. 1 is a diagram schematically illustrating a system for providing a fetal monitoring service, according to an embodiment of the present disclosure.
FIG. 2 is a diagram for describing how a monitoring device obtains movement data and acoustic data, according to an embodiment of the present disclosure.
FIG. 3 is a block diagram of a monitoring device and a service providing server, according to an embodiment of the present disclosure.
FIG. 4 is a flowchart of a method of providing a fetal monitoring service, according to an embodiment of the present disclosure.
FIG. 5 is a diagram illustrating, in a flowchart, processes in which a service providing server generates result data, according to an embodiment of the present disclosure.

### Mode for Invention

Hereinafter, embodiments will be described in detail with reference to the accompanying drawings, and in the following description with reference to the drawings, like reference numerals refer to like elements and redundant descriptions thereof will be omitted.

In addition, various changes may be made to the embodiments, and thus, specific embodiments will be illustrated in drawings and described in detail in the detailed description. Effects and features of the embodiments and methods of achieving the same will become apparent with reference to details described in detail with reference to the drawings. However, the embodiments are not limited to those described below, and may be implemented in various forms.

In the drawings, parts irrelevant to the description are omitted in order to clearly describe the present disclosure, and like reference numerals designate like elements throughout the specification.

In the following embodiments, the terms "first" and "second" are not used in a limited sense and are used to distinguish one component from another component.

In the following embodiments, an expression used in the singular encompasses the expression of the plural, unless it has a clearly different meaning in the context.

In the following embodiments, it will be further understood that the terms "comprise" and/or "comprising" used herein specify the presence of stated features or components, but do not preclude the presence or addition of one or more other features or components.

In the following embodiments, when a part is "connected" to another part, the part may not only be "directly connected" to the other part, but may also be "electrically connected" to the other part with another element in between.

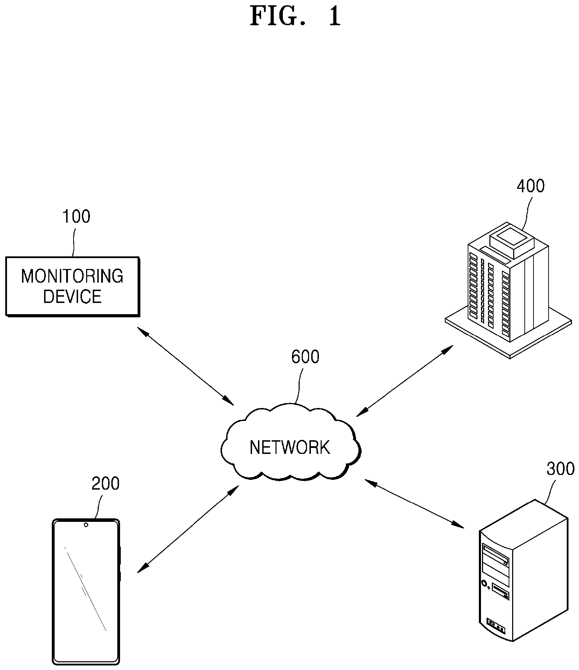

FIG. 1 is a diagram schematically illustrating a system for providing a fetal monitoring service, according to an embodiment of the present disclosure.

Referring to FIG. 1, the system for providing a fetal monitoring service of the present disclosure may include a monitoring device 100, a user terminal 200, a service providing server 300, and a medical system server 400. The monitoring device 100, the user terminal 200, the service providing server 300, and the medical system server 400 may be connected to each other through a network 600 to transmit and receive data. FIG. 1 is an example for describing the present disclosure, and the number of monitoring devices or user terminals or the number of servers is not limited to that shown in FIG. 1.

Users who wish to receive the fetal monitoring service may obtain data related to a status of a fetus through the monitoring device 100. The monitoring device 100 includes at least one sensor module to obtain the data related to the status of the fetus, and may store the data in a memory included in the monitoring device 100 or transmit the data to the service providing server 300 or the medical system server 400.

The user may place the monitoring device 100 around the fetus to obtain the data related to the status of the fetus. The monitoring device 100 may be placed on the abdomen of a pregnant woman to be close to the fetus, and may be in the form of a wearable device that may be worn on the abdomen.

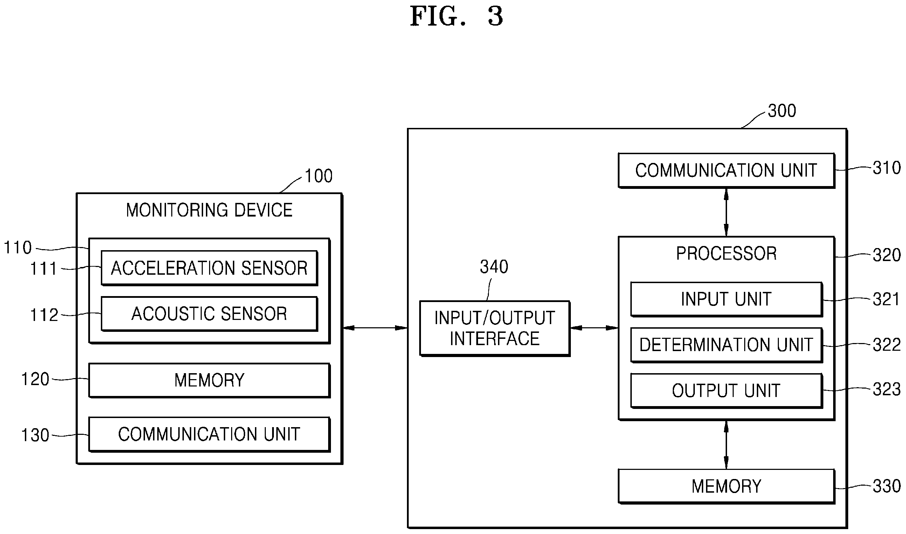

A sensor unit 110 (see FIG. 3) included in the monitoring device 100 may be placed close to the user's abdomen and detect the status of the fetus. Accordingly, the monitoring device 100 may detect movement of the fetus to monitor a fetal status, and obtain raw data about the fetal status.

The sensor unit of the monitoring device 100 may detect movement of the pregnant woman, that is, the user, in addition to the movement of the fetus. Thus, the raw data generated by the monitoring device 100 may include information about the movement of the fetus or the movement of the pregnant woman. The raw data obtained from the monitoring device 100 may be transmitted to the service providing server 300, which will be described below, through the network 600, and be derived as result data related to the status of the fetus. The raw data transmitted to the service providing server 300 may be real-time data or may be pre-stored data.

The user terminal 200 refers to a target device that receives the fetal monitoring service according to the present embodiment. The user terminal 200 may be connected to the monitoring device 100, the service providing server 300, and the medical system server 400 through the network 600 to transmit and receive data.

The user terminal 200 may be a stationary terminal or mobile terminal implemented as a computer device. For example, the user terminal 200 may be implemented as a portable terminal, a wearable terminal, a personal computer (PC), a laptop PC, a tablet PC, a smartphone, or a smart watch, but is not limited thereto, and may be any device capable of transmitting and receiving data to and from a monitoring device and servers and capable of providing a service to a user.

The user terminal 200 may provide, to the user, various pieces of information received from the monitoring device 100, the service providing server 300, and the medical system server 400. In particular, the user terminal 200 may include a user interface that includes information about the status of the fetus and information about the user, provide the result data on the health status of the fetus to the user, and provide a health management service for the fetus and pregnant woman. The method of providing the fetal monitoring service may be implemented by being installing on the user terminal 200 in the form of an application.

In addition, the user may input user information and other information through the user terminal 200. Here, the user information may be information about a pregnant woman, and may include personal information, such as age, height, and contact information, as well as status information, such as weight, body temperature, history of internal and reproductive diseases related to pregnancy, past surgery, and medication use. However, the user information is not necessarily obtained from the user terminal 200, and may also be obtained from the medical system server 400 of a medical institution visited by the user.

The service providing server 300 may be implemented as a computer device or a plurality of computer devices configured to provide a command, code, file, content, and service by communicating with the monitoring device 100, the user terminal 200, and the medical system server 400 through the network 600.

For example, the service providing server 300 may provide a file for installation of an application to the user terminal 200 accessed through the network 600. In this case, the user terminal 200 may install the application by using the file provided by the service providing server 300.

Also, the user may receive a service or content provided by the service providing server 300 by accessing the service providing server 300 according to control by an operating system (OS) or at least one program (e.g., a browser or installed application) included in the user terminal 200. In another example, the service providing server 300 may set a communication session for data transmission and reception, and route the data transmission and reception with the user terminal 200 through the set communication session.

The service providing server 300 performs a function of supporting the fetal monitoring service. In particular, the service providing server 300 may store and manage the user information and other service-related information collected and transmitted from the monitoring device 100, the user terminal 200, and the medical system server 400. In addition, the service providing server 300 may calculate valid data related to the fetus, based on the obtained user information and other service-related information, determine the health status of the fetus therethrough, generate the result data, and provide the result data to the user terminal 200.

Although the user terminal 200 and the service providing server 300 have been described separately, the user terminal 200 and the service providing server 300 may be provided as a single device in some cases. For example, the service providing server 300 may be a configuration within the user terminal 200. Hereinafter, the user terminal 200 and the service providing server 300 will be described as separate devices.

The medical system server 400 is a hospital-side server and may store and manage the user information. The user information may include the personal information of the pregnant woman and the status information of the pregnant woman, and further include the status information of the fetus. The user information stored in the medical system server 400 may be information stored in the medical system server 400 by a medical staff for the pregnant woman.

The medical staff for the pregnant woman may view the user information stored in the service providing server 300 through the medical system server 400. The medical staff for the pregnant woman may analyze the viewed information, make a diagnosis, and store a diagnosis result in the medical system server 400. The pregnant woman may check the diagnosis result through the user terminal 200.

The medical staff for the pregnant woman may store, in the medical system server 400, not only the diagnosis result but also various types of information necessary for the pregnant woman, and the pregnant woman may also check the various types of information through the user terminal 200.

The monitoring device 100, the user terminal 200, the service providing server 300, and the medical system server 400 may be operated by being matched in a one-to-one manner, but the present disclosure is not limited thereto and a one-to-n relationship is possible. In other words, n user terminals 200 may be connected to one monitoring device 100, and one service providing server 300 may be connected to the n user terminals 200. The present disclosure is not limited thereto, and the system for providing the fetal monitoring service may be configured by connecting the monitoring device 100, the user terminal 200, the service providing server 300, and the medical system server 400 in various combinations.

The monitoring device 100, the user terminal 200, the fetal monitoring service providing server 300, and the medical system server 400 of the system for providing the fetal monitoring service, according to an embodiment of the present disclosure, may be connected to the network 600.

A connection method of the network 600 is not limited, and the network 600 refers to a comprehensive data communication network that allows network entities shown in FIG. 1 to communicate smoothly with each other. The communication method of the network 600 may include not only a communication method using a communication network that may be included in the network 600, but also short-range wireless communication between devices.

For example, the network 600 may include a local area network (LAN), a wide area network (WAN), a value-added network (VAN), a mobile radio communication network, a satellite communication network, or a combination thereof, and may include wireless Internet, wired Internet, and a mobile wireless communication network. Examples of wireless communication may include wireless LAN (Wi-Fi), Bluetooth, Bluetooth low energy, ZigBee, Wi-Fi direct (WFD), ultra-wideband (UWB), infrared data association (IrDA), and near field communication (NFC), but are not limited thereto.

Also, the network 600 may include one or more of network topologies including a bus network, a start network, a ring network, a mesh network, a star-bus network, and a tree or hierarchical network, but is not limited thereto.

Through the system for providing the fetal monitoring service described above, the user may continuously monitor the fetus without having to increase the frequency of hospital visit, and may receive a customized and effective health management service for the fetus and pregnant woman.

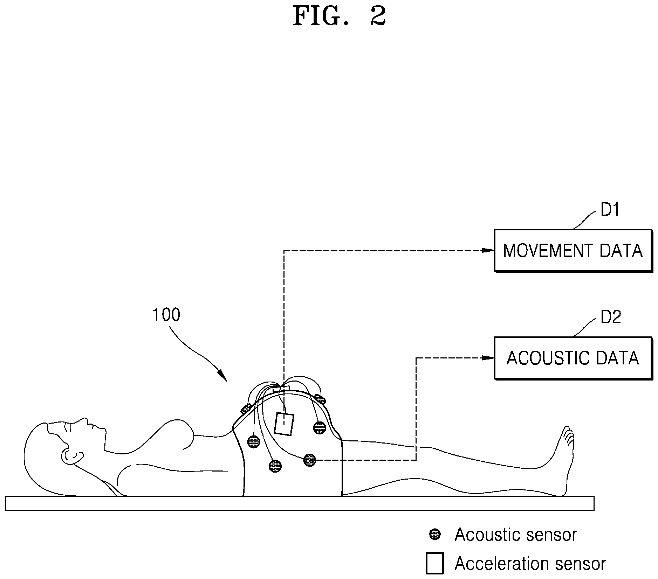

FIG. 2 is a diagram for describing how a monitoring device obtains movement data and acoustic data, according to an embodiment of the present disclosure, and FIG. 3 is a block diagram of a monitoring device and a service providing server, according to an embodiment of the present disclosure.

For the monitoring device 100 and the service providing server 300 shown in FIGS. 2 and 3, only components related to the present embodiment are illustrated to prevent features of the present embodiment from being obscured. Accordingly, it would be obvious to one of ordinary skill in the art that general-purpose components other than the components shown in FIGS. 2 and 3 may be further included.

Referring to FIGS. 2 and 3, the monitoring device 100 according to an embodiment of the present disclosure may include the sensor unit 110, a memory 120, and a communication unit 130.

The monitoring device 100 is a device for detecting the status of the fetus by measuring the movement of the fetus, and the monitoring device 100 may store the raw data detected through the sensor unit 110 in the memory 120 or transmit the raw data to the service providing server 300 through the communication unit 130. The monitoring device 100 may further include a battery (not shown) for operating the device.

The monitoring device 100 may be placed on the abdomen of the user and may be fixed to the abdomen of the user to detect the status of the fetus and transmit the status to the server. For example, the monitoring device 100 may be implemented as a wearable device in the form of a belt that surrounds the abdomen and has an adjustable length for the user to wear. Alternatively, the monitoring device 100 may be implemented in the form of a pad that is formed of an elastic material, includes Velcro on one side, and is adjustable to be closely fit a body size while surrounding the abdomen of the user.

The monitoring device 100, which is shaped to surround the abdomen of the user, may allow the sensor unit 110 to be fixed in close contact with the abdomen of the user, such that the sensor unit 110 more accurately detects the status of the fetus. However, the present disclosure is not limited thereto, and the monitoring device 100 may include the sensor unit 110 including a plurality of sensor pads attachable to the abdomen of the user, and a driving unit connected to the sensor unit 110.

The sensor unit 110 of the monitoring device 100 may detect the status of the fetus and generate the raw data by measuring movement related to the fetus. The sensor unit 110 may include at least one acceleration sensor 111 and an acoustic sensor 112, and each sensor may be arranged to be adjacent to the fetus when the user wears the monitoring device 100.

The acceleration sensor 111 is a sensor for measuring movement as an acceleration value and may be placed on the body of the pregnant woman to detect the position, posture, or movement of the fetus and pregnant woman. The acceleration sensor 111 may be placed in the center of the abdomen of the pregnant woman in order to detect movement of the fetus and pregnant woman, but is not limited thereto. Various types of acceleration sensors 111 may be applied, and for example, an inertial acceleration sensor, a gyro-type acceleration sensor, a silicon semiconductor-type acceleration sensor, or the like may be applied depending on a detection method.

The acceleration sensor 111 may measure the position, posture, or movement of the fetus and pregnant woman, and generate movement data D1 including position information, posture information, and movement information of the fetus and pregnant woman. The movement data D1 may include measurement information about changes in acceleration values caused by torso motion, limb motion, and breathing motion of the fetus, and breathing motion and torso motion of the pregnant woman. For example, the monitoring device 100 may measure the amount of change in each of positions of the head, body, arms, and legs of the fetus by using the acceleration sensor 111, and generate the movement data D1 related to the fetus based the amount of change.

The movement data D1 may include measurement values related to the movement of the pregnant woman in addition to the movement of the fetus. For example, when the user is to monitor the fetus by using the monitoring device 100, even if the measurement is performed without moving from the spot, the movement data D1 detected by the acceleration sensor 111 may include respiration, a cough, muscle contraction, and heartbeat of the pregnant woman. The raw data including the movement data D1 may be transmitted to the service providing server 300 and processed by a processor 320, which will be described below, and may be calculated as the valid data related to the status of the fetus.

The acoustic sensor 112 is a sensor for measuring low-frequency vibration generated by movement or sound, and may be placed on the body of the pregnant woman to detect sound related to the movement of the fetus and pregnant woman or a surrounding stimulus.

A plurality of acoustic sensors 112 may be arranged on the abdomen of the pregnant woman at regular intervals. The plurality of acoustic sensors 112 arranged on the abdomen of the pregnant woman may be linked to each other to generate acoustic data D2 according to the movement of the fetus, through which even minute movement of the fetus may be detected. The number of acoustic sensors 112 arranged on the abdomen of the pregnant woman is not limited to that shown in the drawing, and an appropriate number of acoustic sensors 112 may be used depending on a design.

The acoustic sensor 112 may capture the low-frequency vibration generated by the movement of the fetus and pregnant woman, and the low-frequency vibration may form acoustic data D2 measured in time series. The acoustic data D2 may include measurement information about the low-frequency vibration generated by the torso motion, limb motion, and breathing motion of the fetus, and the breathing motion and torso motion of the pregnant woman. In addition, the acoustic data D2 may include information obtained by measuring other physiological sounds, such as digestive system motion and heart rate motion, which occur in the bodies of the fetus and pregnant woman.

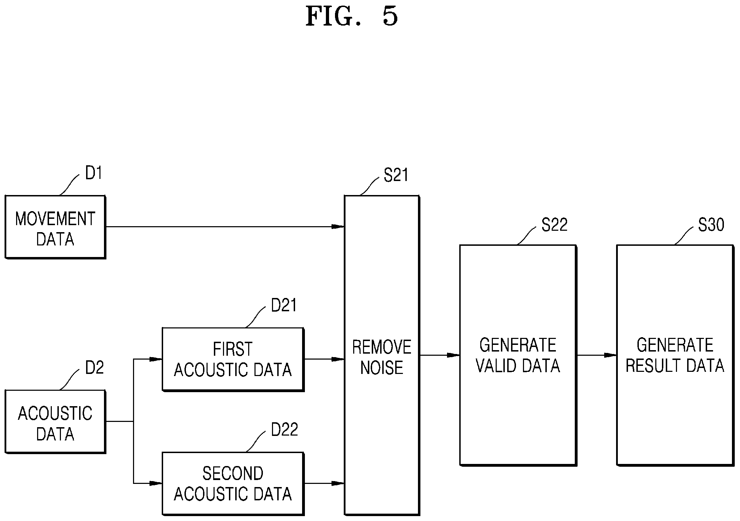

The acoustic sensor 112 may detect sound related to the surrounding stimulus, and the sound related to the surrounding stimulus may configure second acoustic data D22 (see FIG. 5) distinguished from first acoustic data D21 (see FIG. 5) related to the fetus. The second acoustic data D22 related to the surrounding stimulus may be measured by the acoustic sensor 112 arranged on the abdomen of the pregnant woman and then separated from the first acoustic data D21, or may be measured by a separate acoustic sensor (not shown) distinguished from the acoustic sensor 112 and arranged beside the acoustic sensor 112.

The acoustic data D2 obtained from the acoustic sensor 112 constitutes the raw data together with the above-described movement data D1, may be transmitted to the service providing server 300 and processed, and may be calculated as the valid data related to the movement of the fetus.

The memory 120 of the monitoring device 100 may be electrically connected to the sensor unit 110 and may store the raw data measured by the sensor unit 110.

The monitoring device 100 may communicate with the user terminal 200 and/or the service providing server 300 through the communication unit 130. The communication unit 130 may include one or more components enabling communication with the user terminal 200 and/or the service providing server 300. For example, the communication unit 130 may include at least one of a short-range communication unit, a mobile communication unit, and a broadcast reception unit.

The communication unit 130 may transmit the raw data obtained from the monitoring device 100 to the outside, and the raw data may be provided to the service providing server 300. Also, the communication unit 130 may communicate with the user terminal 200 and receive an input signal of the user, transmitted from the user terminal 200. The user may control operations of the monitoring device 100 by using an interface or the like of the user terminal 200. In detail, the user may turn on/off power of the monitoring device 100 by using an interface of an application provided in the user terminal 200, reserve an operating time of the monitoring device 100, and perform control of adjusting sensitivity of a measurement value measured by the sensor unit 110.

The service providing server 300 according to an embodiment of the present disclosure may correspond to at least one processor or may include at least one processor. Accordingly, the service providing server 300 may be driven while being included in a hardware device such as a microprocessor or a general-purpose computer system. Here, the processor may, for example, denote a data processing device embedded in hardware, including a physically structured circuit to perform a function represented by code or command included in a program. Examples of the data processing device embedded in hardware may include processing devices, such as a microprocessor, a central processing unit (CPU), a processor core, a multiprocessor, an application-specific integrated circuit (ASIC), and a field programmable gate array (FPGA), but the scope of the present disclosure is not limited thereto.

The service providing server 300 may be mounted on the user terminal 200 or may be provided as a separate server device. According to another embodiment, the service providing server 300 may include two server devices. In this case, one server may remove noise from the raw data described below and generate the valid data, and the other server may determine the health status of the fetus by using the valid data and generate the result data.

Referring back to FIG. 3, the service providing server 300 according to an embodiment of the present disclosure may include a communication unit 310, a processor 320, a memory 330, and an input/output interface 340.

The communication unit 310 may receive the raw data provided from the monitoring device 100 described above. The communication unit 310 functions as a communication module using wired or wireless communication and may receive the raw data transmitted from the monitoring device 100. Here, the communication unit 310 may include a configuration for communicating with the communication unit 130 of the monitoring device 100 through the network 600 shown in FIG. 1.

The communication unit 310 may communicate with the user terminal 200 or the medical system server 400 in addition to the monitoring device 100. The communication unit 310 may receive the user information transmitted from the user terminal 200 or the medical system server 400. Also, the communication unit 310 may provide the result data processed by the processor 320 to the user terminal 200 or the medical system server 400.

The processor 320 may be configured to process a command of a computer program by performing basic arithmetic, logic, and input/output operations. The command may be provided to the processor 320 by the memory 330 or the communication unit 310. For example, the processor 320 may be configured to execute a received command according to a program code stored in a recording device, such as the memory 330.

The processor 320 may control all operations of the service providing server 300. For example, the processor 320 may execute a program stored in the memory 330 to control the communication unit 310 and the memory 330 in general.

The processor 320 may include an input unit 321, a determination unit 322, and an output unit 323. The processor 320 may generate the result data related to the health status of the fetus by processing the raw data received from the monitoring device 100 through the input unit 321, the determination unit 322, and the output unit 323.

The input unit 321 may obtain, in time series, the raw data including the movement data D1 related to the fetus and pregnant woman, the movement data D1 generated by the monitoring device 100, and the acoustic data D2 related to the fetus and surrounding stimulus. The movement data D1 and the acoustic data D2 may be provided to the input unit 321 by being linked to each other by the monitoring device 100 in time series, or on the other hand, the input unit 321 may separately receive the movement data D1 and the acoustic data D2 from the monitoring device 100 and perform an operation of linking the pieces of data in time series.

The determination unit 322 may remove noise unrelated to the movement or status of the fetus from the time-series linked raw data and generate the valid data by matching the same with the pre-stored user information. For example, raw data may include user movement that is unrelated to fetal movement. The determination unit 322 may calculate the valid data related to the movement or status of the fetus, from the movement data D1 and the acoustic data D2, which are linked to each other in time series by using time series information and user information.

In particular, the determination unit 322 may track the position and movement of the fetus and calculate the valid data related to the movement of the fetus. Here, in order to calculate the valid data, the determination unit 322 may match pieces of characteristic information of the raw data with the pre-stored user information.

The valid data generated by the determination unit 322 may include information about the position and movement of the head of the fetus and the position and movement of the limbs of the fetus. Also, the valid data may include information about movement of the fetus related to the fetal heartbeat, movement of the fetus related to an external sound stimulus, and movement of the fetus related to an external physical stimulus. The information about the movement of the fetus, included in the valid data, may include information about the range, frequency, speed, rotation angle, and change in motion of movement of each body part of the fetus.

The output unit 323 may determine the health status of the fetus by using the valid data and generate the result data based on the health status of the fetus. For example, the output unit 323 may determine the health status of the fetus by using the valid data related to the movement of the fetus, and status information and development information of the fetus and status information of the pregnant woman, which are stored in the memory 330. The output unit 323 may calculate the determination on the health status of the fetus as the result data and transmit the result data to the outside.

For example, the output unit 323 may specify the position and posture of the fetus, based on the head of the fetus, by using information related to movement of the head of the fetus, included in the valid data, and determine how long or how often the fetus stayed at which position in a space spanning the abdomen and pelvis of the pregnant woman. In addition, the output unit 323 may determine a main connection posture and motion of the torso and limbs of the fetus, based on the head of the fetus, and determine in which direction the face of the fetus is turned.

In another example, the output unit 323 may specify positions and bent postures of the hands, feet, arms, and legs of the fetus by using information related to movement of the limbs of the fetus, included in the valid data, and determine bending angles of the arms and legs, a bending posture of the finger, and a motion of each body part.

The output unit 323 may determine the health status of the fetus according to a development status of the fetus, based on the movement of the fetus determined as described above, and generate the result data including the health status of the fetus. For example, the output unit 323 may determine the amount (too little or too much), speed (slow or fast), or occurrence of tremors in overall movement of the fetus, based on movement of the entire body of the fetus and motion statuses of proximal limbs (trunk, shoulder and hip, and elbows and knees) and distal limbs (wrists, ankles, and fingers), and generate the result data related to the health status of the fetus.

Meanwhile, the output unit 323 may generate the result data related to the health status of the fetus by determining movement of the fetus, which occurs when an external stimulus is applied. For example, the output unit 323 may generate the result data related to the health status of the fetus by determining a change in movement of the fetus when an external sound stimulus (e.g., voice of the pregnant woman) is applied or when an external physical stimulus (e.g., a stimulus of the pregnant woman touching the stomach with a hand) is applied. Also, the output unit 323 may generate the result data related to the health status of the fetus by linking heartbeat information of the fetus, monitored by the monitoring device 100, with data about the movement of the fetus.

As described above, the output unit 323 may determine the health status of the fetus by using data including the range, frequency, speed, changes in motion type and posture, and a change in motion of movement of each body part of the fetus, and generate the result data including information about the health status of the fetus. Here, the result data may include data matched to the status information and development information of the fetus, and the status information of the pregnant woman. For example, the result data may include information about a motion developmental delay of the fetus, which is determined based on the development information of the fetus and an overall motion status of the fetus, and may include information about hemiplegia determined based on movement of the limbs on one side of the fetus, and information about brain exercise development abnormalities determined based on determining that tremors or excessive motion has occurred in a body part of the fetus.

In addition, the result data may include determination information about torticollis and plagiocephaly in the fetus by determining whether the position of the fetus is mainly fixed to the abdomen of the pregnant woman, based on the movement of the fetus. Also, the result data may include information about determining torticollis, a difference in tension between two limbs, and sensory integration abnormalities of the fetus by determining a change in positions of the trunk and head of the fetus, based on the direction of gravity, and an overall amount of motions.

The result data may include health information related to a current status of the fetus, may also include predictive information about a future health and development status of the fetus, and may include health information related to a status of the fetus after birth.

The result data may be generated based on pieces of data regularly measured by the user by using the monitoring device, but is not limited thereto and may be generated based on randomly measured data.

Then, the result data may be used to compare and analyze infant treatment information related to the health status of the fetus after birth, such as a head shape, presence of torticollis, motor and cognitive development, and social development after the birth of the fetus. Accordingly, the server for providing the fetal monitoring service may derive an algorithm for predicting the health status of the fetus after birth from the result data, and may predict the health status of the fetus after birth by applying the result data to the algorithm.

For example, the result data may be used to predict possibilities of various diseases in the fetus after birth, such as autism spectrum disorder, intellectual disability, cerebral palsy, various genetic diseases, torticollis, head asymmetry, scoliosis, muscle disease, and motor neuron disease. Also, the result data may be used to predict a risk of delivery for the pregnant woman and may provide information necessary to determine a cesarean section. Accordingly, an embodiment of the present disclosure may provide a service for early discovery of various diseases and provide a customized service to improve the health of a fetus and pregnant woman.

Pieces of data derived by the service providing server 300 may be used as training data for determining the health status of the fetus. The service providing server 300 may increase accuracy of monitoring the status of the fetus through repetitive learning and may derive accurate result data related to the current health status of the fetus. In addition, the service providing server 300 that has been repeatedly trained may predict the possibilities of various diseases that may occur in the fetus with high accuracy by increasing the accuracy of monitoring the status of the fetus, and derive the result data related to the future health status. Accordingly, a method of providing a fetal monitoring service of the present disclosure may provide a customized health management service tailored to the health status of the fetus and promote the health of the fetus and pregnant woman.

The memory 330 is a computer-readable recording medium, and may include random access memory (RAM), read-only memory (ROM), and a permanent mass storage device such as a disk drive. Also, the memory 330 may store an OS and at least one program code (for example, code for a browser or the application installed and driven in the user terminal 200). Such software components may be loaded from a computer-readable recording medium separate from the memory 330, by using a drive mechanism. Such a separate computer-readable recording medium may include a computer-readable recording medium such as a floppy drive, a disk, a tape, a DVD/CD-ROM drive, or a memory card. According to another embodiment, the software components may be loaded on the memory 330 through the communication unit 310, instead of the computer-readable recording medium. For example, at least one program may be loaded on the memory 330 based on a program (e.g., the application described above) installed by files provided by developers or a file distribution system distributing an application installation file, through the network 600.

At least one program code for controlling the monitoring device 100, the user terminal 200, the service providing server 300, or the medical system server 400 may be stored in the memory 330. Also, at least one program code for generating the result data from the raw data transmitted from the monitoring device 100 may be stored.

The memory 330 may store and manage the raw data transmitted from the monitoring device 100, and the valid data, the result data, and the user information generated by the service providing server 300. When the service providing server 300 is connected to a plurality of user terminals, the memory 330 may compress and encode data for each user, and store and manage the same.

The input/output interface 340 may be a unit for an interface with an input/output device. For example, an input device may include a device such as a keyboard or a mouse, and an output device may include a device such as a display for displaying a communication session of an application. In another example, the input/output interface 340 may be a unit for an interface with a device in which functions for input and output are integrated, such as a touch screen. In detail, with respect to the processor 320 of the service providing server 300 processing a command of a computer program loaded on the memory 330, a service screen or content configured by using data provided by the user terminal 200 may be displayed on a display via the input/output interface 340.

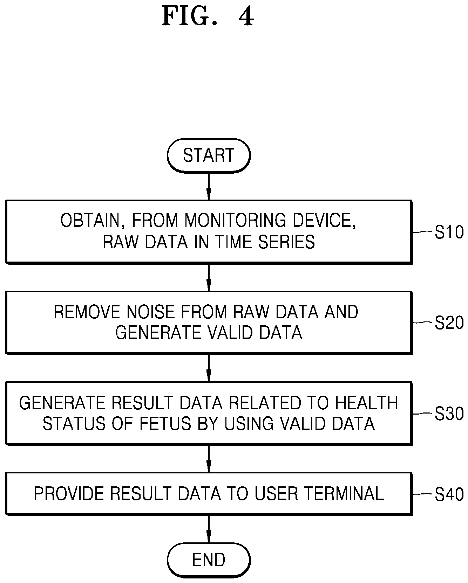

FIG. 4 is a flowchart of a method of providing a fetal monitoring service, according to an embodiment of the present disclosure, and FIG. 5 is a diagram illustrating, in a flowchart, a process in which a service providing server generates result data, according to an embodiment of the present disclosure.

Referring to FIGS. 4 and 5, the method of providing a fetal monitoring service, according to an embodiment of the present disclosure, may include obtaining, from a monitoring device, raw data in time series (operation S10), removing noise from the raw data and generating valid data (operation S20), generating result data related to a health status of a fetus by using the valid data (operation S30), and providing the result data to a user terminal (operation S40).

Hereinafter, each operation of the method of providing a fetal monitoring service, according to an embodiment of the present disclosure, will be described in further detail.

First, the method of providing a fetal monitoring service, according to an embodiment of the present disclosure, obtains, from the monitoring device, the raw data related to the fetus and pregnant woman in time series (operation S10).

The raw data related to the fetus and pregnant woman provided from the monitoring device may include movement data and acoustic data generated based on measurement values sensed by at least one of an acceleration sensor and an acoustic sensor of the monitoring device.

The monitoring device may be placed on the abdomen of the pregnant woman and measure positions, postures, or movements of the fetus and pregnant woman during a certain period of time. In this case, the positions, postures, or movements of the fetus and pregnant woman measured by the acceleration sensor may form the movement data, and vibrations related to the fetus and surrounding stimulus measured by the acoustic sensor may form the acoustic data. The monitoring device may generate the raw data by combining the movement data and the acoustic data in time series.

The monitoring device may transmit the generated raw data to the service providing server through a communication unit included in the monitoring device. For example, the monitoring device may generate and store the raw data and then transmit the raw data to the service providing server according to a determined schedule, or receive a user's request using the user terminal and transmit the raw data to the service providing server. Alternatively, the raw data may be data transmitted by being measured in real time by the monitoring device.

The service providing server may receive the raw data transmitted from the monitoring device through the communication unit of the service providing server and transmit the raw data to an input unit of a processor. Accordingly, the processor may obtain, from the monitoring device in time series, the raw data including the movement data related to the fetus and pregnant woman and the acoustic data related to the fetus and surrounding stimulus.

Then, noise is removed from the raw data by using time series information of the raw data and the valid data is generated by matching the noise-removed raw data with pre-stored user information (operation S20).

The raw data received through the communication unit of the service providing server may undergo an arithmetic process in the processor for calculating a valid value. The raw data includes, in addition to data related to the fetus, data related to an external stimulus or movement of the user which is unrelated to movement or status of the fetus, and thus, a process of removing noise to calculate the data related to the fetus is required.

The service providing server may include, in the processor, at least one program for removing noise, thereby removing noise from the raw data and generating the valid data.

The removing of noise (operation S21) may include removing noise values that are not related to the fetus, from the movement data and acoustic data measured by the monitoring device. At this time, comparison with a pre-measured data value may be performed to specify noise, or noise may be removed by using a learned algorithm.

Then, the valid data is generated by using noise-removed data (operation S22).

The valid data may include the data related to the fetus. In detail, the valid data may be data in which noise-removed movement data, first acoustic data, and second acoustic data are matched to pre-stored user information. Here, the user information may be matched to the movement data, the first acoustic data, and the second acoustic data after an encoding process.

Then, a health status of the fetus is determined by using the valid data and the result data is generated (operation S30).

The valid data from which noise has been removed may include information related to the status or movement of the fetus, and accordingly, the service providing server may determine the health status of the fetus and generate the result data.

The service providing server may generate the result data by using the movement data, the first acoustic data, and the second acoustic data, from which noise is removed and which are linked to each other in time series. Here, the service providing server may extract characteristic information from each piece of data, determine the status or movement of the fetus by using the characteristic information extracted at a same time, and use the determined status information of the fetus to generate the result data.

According to an embodiment, the service providing server may extract characteristic information about ambient noise occurring at a specific time by using the movement data matched to the second acoustic data corresponding to ambient sound, and match the characteristic information to fetal movement information at the same time to determine whether the fetus moves normally in response to a sound stimulus.

For example, the service providing server may extract following status information of the fetus from the valid data:
Determination of a position of an axis of a head of a fetus relative to gravity (a left side or a right side relative to gravity); where a head of a fetus stays the longest time when a womb is divided into quadrants; how often a fetus moves inside a womb; how often a fetus rotates based on a head-torso axis; whether a head of a fetus is positioned more in relation to a torso (left turn-right turn, head down-tilted back, tilted left or right); a speed, direction, and range of torso movement of a fetus and an amount (frequency) of the movement; a speed, range, and direction of movement of shoulder joints, hip joints, elbow joints, and knee joints on both sides of a fetus and an amount (frequency) of the movement; a most prominent angle of flexion of wrists and ankles of a fetus, and a speed and amount of movement thereof; determination of a speed variation status, amplitude, and presence of tremor when a fetus moves from a head to a pelvis, from both shoulder joints to fingers, and from both hip joints to ankles; measurement of a motion occurrence situation, such as Moro reflex triggered by an external stimulus; measurement of changes in movement patterns of left and right upper and lower extremities according to movement (rotation and bending) of a head and a change in position relative to a direction of gravity (neck righting reaction, asymmetric tonic neck reflex occurrence, and the like); and identification of a change pattern of movement of a fetus according to a status of a pregnant woman (sleep, eating, exercise, stress, or the like), time, temperature, and surrounding sound stimulus, and general movement (method according to Pretchl's classification).

When the status information described above is derived, the service providing server may determine the health status of the fetus from the status information and generate the result data. The result data is data related to monitoring of the fetus, provided to the user through the user terminal, and may be data calculated by at least one program performing an operation on the health status of the fetus, based on the valid data. The result data may also include determination on the health status of the fetus that is additionally input by a medical staff of the user.

In addition, the result data may further include lifestyle guides for promoting health of the fetus and pregnant woman, based on results of at least one program determining the health status of the fetus. The lifestyle guides may include an exercise program, an eating habits program, and a sleep program, and may be derived based on a program stored in the service providing server or may be provided by the medical staff through a medical system server.

Then, the result data is provided to the user terminal (operation S40).

The service providing server may transmit the generated result data to the user terminal or medical system server. The user may identify the result data by using the user terminal, and the result data provided to the user may further include explanatory content for the user to understand the result data.

Embodiments of the present disclosure may promote health of a pregnant woman and fetus throughout the entire period of pregnancy and childbirth without having to increase a frequency of hospital visits, by enabling a user and a medical staff of the user to conveniently monitor a status of the fetus. In addition, a method of providing a fetal monitoring service of the present disclosure may provide a health management service suitable for a user, based on fetal monitoring results, and may greatly improve user convenience.

The embodiments may also be realized in the form of a recording medium including instructions executable by a computer, such as a program module executed by a computer. A computer-readable medium may be an arbitrary available medium accessible by a computer, and includes all volatile and non-volatile media and separable and non-separable media. Further, examples of the computer-readable recording medium may include a computer storage medium and a communication medium. Examples of the computer storage medium include all volatile and non-volatile media and separable and non-separable media, which have been implemented by an arbitrary method or technology, for storing information such as computer-readable instructions, data structures, program modules, and other data. The communication medium typically includes a computer-readable instruction, a data structure, a program module, other data of a modulated data signal, or another transmission mechanism, and an example thereof includes an arbitrary information transmission medium.

Furthermore, in the specification, the term "unit" may be a hardware component such as a processor or circuit and/or a software component that is executed by a hardware component such as a processor.

Hereinabove, the present disclosure has been described with reference to the embodiments shown in the drawings, but the embodiments are only examples and it would be understood by one of ordinary skill in the art that various modifications and equivalent embodiments are possible. Accordingly, the scope of the present disclosure will be defined by the appended claims.

### Explanation Of Reference Numerals Designating The Major Elements Of The Drawings

- 100:: Monitoring Device
- 200:: User Terminal
- 300:: Fetal Monitoring Service Providing Server
- 400:: Medical System Server

## Claims

1. A method of providing a fetal monitoring service, the method comprising:
obtaining, from a monitoring device in time series, raw data including movement data related to a fetus and a pregnant woman and acoustic data related to the fetus and a surrounding stimulus;
removing noise from the raw data by using time series information and generating valid data by matching the noise-removed raw data with pre-stored user information;
determining a health status of the fetus by using the valid data and generating result data; and
providing the result data to a user terminal.

2. The method of claim 1, wherein the acoustic data comprises first acoustic data related to the fetus and second acoustic data related to ambient sound excluding the fetus.

3. The method of claim 2, wherein the determining of the health status of the fetus and the generating of the result data comprises determining the health status of the fetus by linking, in time series, the movement data, the first acoustic data, and the second acoustic data.

4. The method of claim 1, wherein the movement data comprises acceleration data sensed by an acceleration sensor of the monitoring device.

5. The method of claim 1, wherein the user information comprises personal information of the pregnant woman and status information of the pregnant woman.

6. A computer-readable recording medium storing a program for executing the method of any one of claims 1 to 5.

7. A service providing server comprising:
a memory storing at least one program;
a communication unit configured to transmit and receive data to and
from a monitoring device for measuring a status of a fetus, a user terminal, and a medical system server; and
at least one processor configured to provide a fetal monitoring service to a user by executing the at least one program,
wherein the memory includes instructions that cause the at least one processor to:
obtain, from the monitoring device in time series, raw data including movement data related to a fetus and a pregnant woman and acoustic data related to the fetus and a surrounding stimulus;
remove noise from the raw data by using time series information and generate valid data by matching the noise-removed raw data with pre-stored user information;
determine a health status of the fetus by using the valid data and generate result data; and
provide the result data to the user terminal.
